(19) Europäisches Patentamt
European Patent Office
Office européen des brevets

(11) Veröffentlichungsnummer: **0 051 240**
**B1**

(12) # EUROPÄISCHE PATENTSCHRIFT

(45) Veröffentlichungstag der Patentschrift: **10.10.84**

(51) Int. Cl.³: **C 07 C 49/307**, C 07 C 49/547, C 07 C 45/67, C 07 C 45/45, C 07 C 45/82, C 11 B 9/00

(21) Anmeldenummer: **81108917.6**

(22) Anmeldetag: **26.10.81**

(54) **Acetylcycloundecan und Acetylcycloundecene, deren Verwendung und diese enthaltende Riechstoffkompositionen.**

(30) Priorität: **03.11.80 DE 3041250**

(43) Veröffentlichungstag der Anmeldung: **12.05.82 Patentblatt 82/19**

(45) Bekanntmachung des Hinweises auf die Patenterteilung: **10.10.84 Patentblatt 84/41**

(84) Benannte Vertragsstaaten: **AT BE CH DE FR GB IT LI NL**

(56) Entgegenhaltungen:
**FR - A - 2 242 360**
**FR - A - 2 357 514**

**chemical abstracts, Band 89, 1978, Seite 554, Zusammenfassung 214969j, COLUMBUS OHIO (US), S.N. ANFILOGOVA et al.: "Acylation of seven-to-twelve membered cycloolefins"**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Upadek, Horst, Dr., Kempenweg 18A, D-4006 Erkrath (DE)**
Erfinder: **Bruns, Klaus, Dr., Notburgaweg 6, D-4150 Krefeld-Traar (DE)**

**EP 0 051 240 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind Acetylcycloundecan der Formel I und Actetylcycloundecene der allgemeinen Formel II

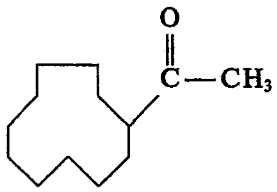

(I)

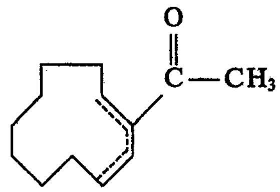

(II)

in der jeweils eine der gestrichelt gezeichneten Bindungen eine Doppelbindung bedeutet, sowie das Gemisch der drei isomeren Acetylcycloundecene der allgemeinen Formel II. Es wurde gefunden, daß diese Verbindungen neue, wertvolle Riechstoffe sind.

Bisher sind nur die homologen Acetylcyclododecene aus der deutschen Offenlegungsschrift 2 630 835 bekanntgeworden, die aber nach der genannten Publikation einen moschusartigen Geruchscharakter besitzen. Die erfindungsgemäßen Acetylcycloundecene unterscheiden sich in ihren Geruchsnoten überraschenderweise deutlich von diesen Verbindungen und zeichnen sich durch holzige Ionon-Ambra-Duftnoten aus.

Die Herstellung der erfindungsgemäßen neuen Riechstoffe erfolgt nach an sich bekannten Syntheseverfahren der organischen Chemie. Ausgangsmaterial für die Synthese des Acetylcycloundecans ist die käufliche Cycloundecancarbonsäure (III), deren Herstellung z. B. von W. Ziegenbein in Chem. Ber. 94 (1961), S. 2989—2992, sowie in der französischen Patentschrift 1 264 032 und in der britischen Patentschrift 989 476 beschrieben wird.

Durch Überführung der Cycloundecancarbonsäure (III) in das Säurechlorid (IV) mittels Thionylchlorid, Umsetzung mit Natriummalonsäuredialkylester in Toluol unter Bildung des entsprechenden Acylmalonesters (V) und Hydrolyse unter gleichzeitiger Decarboxylierung in einer Mischung aus Essigsäure und Schwefelsäure erhält man das Acetylcycloundecan (I)

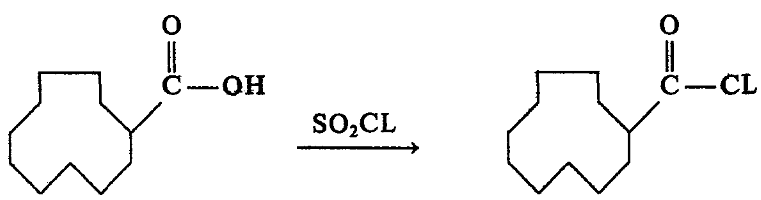


(III) (IV)

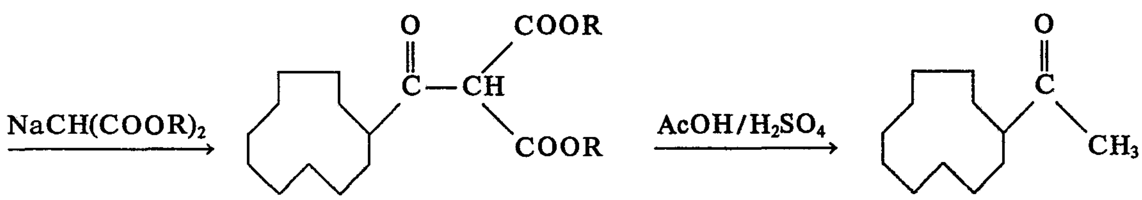


(V) (I)

in sehr reiner Form und hoher Ausbeute.

Ein alternativer, einstufiger Syntheseweg ausgehend von der bekannten Cycloundecancarbonsäure (III) durch Umsetzung mit zwei Äquivalenten Methyllithium in Diethylether erweist sich als ebenfalls gangbar. Dieses einstufige Verfahren wurde z. B. für die Synthese der Acetylcycloundecene (II) gewählt. Ausgangsmaterial ist die 1-Cycloundecencarbonsäure (VI), die nach W. Ziegenbein (Chem. Ber. 94, 1961, S. 2981—2992) leicht aus $\alpha,\alpha'$-Dibromcyclododecanon durch Behandeln mit wäßrigem oder alkoholischem Alkalihydroxyd zugänglich ist.

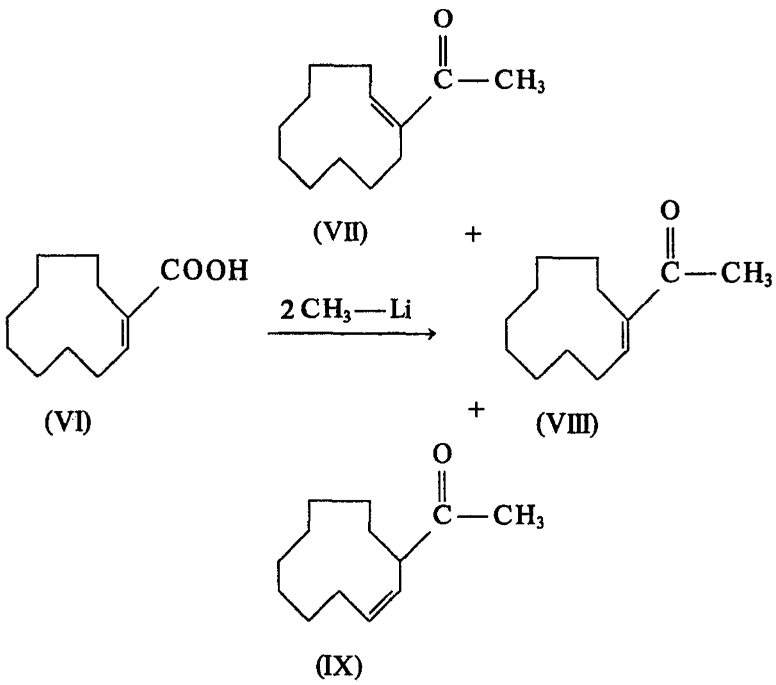


(VII) + (VI) (VIII) + (IX)

Umsetzung der Säure VI mit zwei Äquivalenten Methyllithium in Diethylether ergibt nach Aufarbeitung und Destillation ein Gemisch der Isomeren Acetylcycloundecene VII, VIII und IX, in welchem diese im Verhältnis 2,2 (Z-1-Acetyl-1-cycloundecen, VII) : 1,8 (E-1-Acetyl-1-cycloundecen, VIII) : 1,0 (1-Acetyl-2-cycloundecen, IX) vorliegen.

Das so erhaltene Gemisch der Acetylcycloundecene kann direkt als Riechstoff verwendet werden. Es gelingt jedoch auch, durch Feinfraktionierung die Isomeren Z-1-Acetyl-1-cycloundecen (VII) und E-1-Acetyl-1-cycloundecen (VIII) abzutrennen. Die Isomeren können auf diese Weise auch einzeln als Riechstoffe zur Anwendung kommen.

Durch Behandeln des Isomerengemisches VII—IX mit katalytischen Mengen einer alkoholischen Natriumalkoholatlösung bei 60—70° C kann dieses in das einheitliche Isomere E-1-Acetyl-1-cycloundecen (VIII) überführt werden.

Die erfindungsgemäßen neuen Riechstoffe zeichnen sich durch kräftige, holzig-erdige Ionon-Ambra-Geruchsnoten aus und lassen sich vorzüglich mit anderen, natürlichen und synthetischen Riechstoffen in verschiedenen Mengenverhältnissen mischen und zu neuen, interessanten Duftkompositionen kombinieren. Im allgemeinen liegt ihr Anteil in Riechstoffkompositionen im Bereich von 1 bis 50 Gewichtsprozent, bezogen auf die gesamte Komposition. Derartige Kompositionen können zur Parfümierung von kosmetischen Produkten wie Duftwässern, Aerosolpräparaten, Badepräparaten, Shampoos, Cremes, Lotionen und Toilettenseifen sowie auch in der Extrait-Parfümerie verwendet werden. Sie können aber auch zur Geruchsverbesserung chemisch-technischer Produkte, wie Wasch- und Reinigungsmittel, Wäscheweichspülmittel, Textilbehandlungsmittel, eingesetzt werden. Zur Parfümierung der verschiedenen Produkte werden diesen die Riechstoffkompositionen im allgemeinen in Konzentrationen von 0,05 bis 2 Gewichtsprozent, bezogen auf das gesamte Produkt, zugesetzt.

Die nachfolgenden Beispiele sollen den Gegenstand der Erfindung näher erläutern, ohne ihn hierauf zu beschränken.

## Beispiele

### Beispiel 1

#### Herstellung von Acetylcycloundecan (I)

19,8 g (0,1 Mol) Cycloundecancarbonsäure wurden in 50 ml trockenem Cyclohexan vorgelegt und tropfenweise mit 13,1 g (0,11 Mol) Thionylchlorid, gelöst in 30 ml Cyclohexan, unter Stickstoffatmosphäre versetzt. Es wurde bis zur Beendigung der Gasentwicklung erhitzt. Nach Entfernung des überschüssigen Thionylchlorids und des Cyclohexans im Wasserstrahlvakuum wurde das rohe Cycloundecancarbonsäurechlorid (IV) erhalten.

3,5 g (0,15 Mol) Natrium und 26,4 g (0,2 Mol) Malonsäuredimethylester wurden in 450 ml trockenem Toluol unter Stickstoffatmosphäre bis zur vollständigen Lösung des Metalls erhitzt. Nach Abkühlung auf Raumtemperatur wurde zu der dickflüssigen, aber noch rührfähigen Suspension das rohe Cycloundecancarbonsäurechlorid (20 g, 0,93 Mol), gelöst in 150 ml Toluol, innerhalb 20 Minuten getropft. Man ließ 2,5 Stunden bei Raumtemperatur und 1 Stunde unter Rückfluß nachrühren.

Die Reaktionsmischung wurde unter Kühlung mit 2-n-Salzsäure neutralisiert. Die organische Phase wurde abgetrennt, eingeengt und im Ölpumpenvakuum von Resten flüchtiger Bestandteile befreit. Der so erhaltene rohe Acylmalonester (V) wurde mit einer Lösung von 30 ml Essigsäure, 3,75 ml Schwefelsäure und 20 ml Wasser versetzt und 3 Stunden unter Rückfluß gerührt. Nach Abdestillation des gebildeten Methanols wurde nochmals 2 Stunden bis zur Beendigung der $CO_2$-Entwicklung erhitzt. Die abgekühlte Mischung wurde mit 30 ml 50%iger Natronlauge alkalisch gestellt und mit Ether extrahiert. Die vereinigten organischen Phasen wurden neutral gewaschen, über Magnesiumsulfat getrocknet und eingeengt. Das Rohprodukt (16,8 g) wurde im Ölpumpenvakuum destilliert. Bei 84° C/0,5 mbar wurden 12,9 g Acetylcycloundecan mit $n_D^{20} = 1{,}4828$ isoliert.

IR (Film): 1710 $cm^{-1}$ (C=O), 1355 $cm^{-1}$ ($-CO-CH_3$).
$^1$H-NMR ($CDCl_3$): u. a. $\delta = 2{,}06$ ppm (s, 3H, $-CO-CH_3$).

Geruch: Ionon-Ambra-Note.

## Beispiel 2

### Herstellung des Isomerengemisches der Acetylcycloundecene (II)

Zu 12 g (0,061 Mol) 1-Cycloundecencarbonsäure in 160 ml trockenem Ether wurden unter Feuchtigkeitsausschluß 80 ml einer 1,6molaren Lösung von Methyllithium (0,128 Mol) in Ether unter Eiskühlung bei 10—20° C in $^1/_2$ Stunde zugetropft. Es wurde 3 Stunden bei Raumtemperatur nachgerührt, die Mischung auf Eiswasser gegossen und dreimal mit Ether extrahiert. Die Ether-Phasen wurden vereinigt, neutral gewaschen, über Magnesiumsulfat getrocknet und eingeengt. Durch Destillation wurden bei 92—95° C/1,6 mbar 6,1 g eines Produktes vom Brechungsindex $n_D^{20} = 1{,}4986$ gesammelt. Nach IR/$^1$H-NMR/GC-Analyse handelt es sich um ein etwa 2,2 : 1,8 : 1,0-Gemisch von Z-1-Acetyl-1-Cycloundecen, E-1-Acetylcycloundecen und 1-Acetyl-2-cycloundecen.

Geruch: Holz-, Ambra-Note.

## Beispiel 3

### Herstellung des reinen Z-1-Acetyl-1-cycloundecen (VII)

Durch Feinfraktionierung des in Beispiel 2 erhaltenen Produktgemisches konnte bei 88° C/1,3 mbar reines Z-1-Acetyl-1-cycloundecen mit $n_D^{20} = 1{,}4998$ gewonnen werden.

IR (Film): u. a. 1691 (C=O), 1630 (C=C), 1350 $\left(-\overset{\overset{\displaystyle O}{\|}}{C}-CH_3\right)$ $cm^{-1}$.

$^1$H—NMR ($CDCl_3$): $\delta = 5{,}80$ (t, 1 H, C=C—H), 2,30 (m, 4 H, allylische Protonen), 2,23 s, 3 H, $\left(-\overset{\overset{\displaystyle O}{\|}}{C}-CH_3\right)$, 1,75—1,10 (m, 14 H) ppm.

Geruch: holzig, Ionon-, Ambra-Note.

## Beispiel 4

### Herstellung des reinen E-1-Acetyl-1-cycloundecen (VIII)

a) Reines E-1-Acetyl-1-cycloundecen destillierte aus dem in Beispiel 2 erhaltenen Isomerengemisch bei 98° C/1,3 mbar mit $n_D^{20} = 1{,}5018$ über.

IR (Film): u. a. 1672 (C=O), 1632 (C=C), 1351 $\left(-\overset{\displaystyle O}{\overset{\|}{C}}-CH_3\right)$ $cm^{-1}$.

$^1$H–NMR ($CDCl_3$): $\delta = 6{,}52$ (t, 1 H, C=C–H), 2,40 (m, 4 H, allylische Protonen),

2,30 s, 3 H, $\left(-\overset{\displaystyle O}{\overset{\|}{C}}-CH_3\right)$, 1,85–1,10 (m, 14 H) ppm.

Geruch: holzig, erdig, Kokos-Note.

b) Durch 60minütiges Rühren bei 65° C von 2 g des Produktgemisches II mit 0,2 g Natriummethylat, als 30%ige Lösung in Methanol, wurden nach üblicher Aufarbeitung und Kurzwegdestillation 1,5 g E-1-Acetyl-1-cycloundecen mit den unter a) angegebenen spektralen Daten erhalten.

## Beispiel 5

### Nostalgischer Komplex für Extrait bzw. Eau de Cologne

| | |
|---|---|
| Acetylcycloundecan | 75 Gewichtsteile |
| Cyclamber, Henkel | 75 Gewichtsteile |
| Ambroxan 1% i. DEP | 10 Gewichtsteile |
| $\alpha$-Isomethyljonon | 80 Gewichtsteile |
| Bergamottöl | 60 Gewichtsteile |
| Benzylsalicylat | 50 Gewichtsteile |
| Hydroxyzitronellal | 50 Gewichtsteile |
| $\alpha$-Hexylzimtaldehyd | 40 Gewichtsteile |
| Isoeugenol | 40 Gewichtsteile |
| Moschus Ambrette | 40 Gewichtsteile |
| Moschus Keton | 40 Gewichtsteile |
| Phenylethylalkohol | 40 Gewichtsteile |
| Sandelholzöl ostindisch | 40 Gewichtsteile |
| Vetiverylacetat | 40 Gewichtsteile |
| Ylang-Ylang extra | 40 Gewichtsteile |
| Aldehyd C 14 sog. 10% i. DEP | 30 Gewichtsteile |
| Benzoe Siam Resinoid | 30 Gewichtsteile |
| Eugenol | 30 Gewichtsteile |
| Styrax Resinoid | 30 Gewichtsteile |
| Amylsalicylat | 20 Gewichtsteile |
| Benzylacetat | 20 Gewichtsteile |
| $\delta$-Dekalacton 10% i. DEP | 20 Gewichtsteile |
| Geraniol | 20 Gewichtsteile |
| Jasmin abs. 10% i. DEP | 20 Gewichtsteile |
| Castoreum abs. 10% i. DEP | 15 Gewichtsteile |
| Cyclopentadekanolid | 10 Gewichtsteile |
| Patchouliöl | 10 Gewichtsteile |
| Undecylenaldehyd 10% i. DEP | 10 Gewichtsteile |
| Civette abs. 10% i. DEP | 5 Gewichtsteile |
| Eichenmoos abs. grün | 5 Gewichtsteile |
| Zimtöl Ceylon 10% i. DEP | 5 Gewichtsteile |
| | 1000 Gewichtsteile |

**Patentansprüche**

1. Acetylcycloundecan der Formel I und Acetylcycloundecene der allgemeinen Formel II

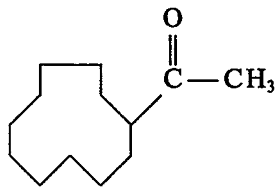

(I)

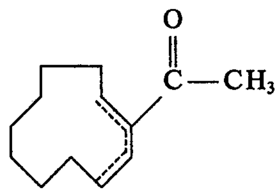

(II)

in der jeweils eine der gestrichelt gezeichneten Bindungen eine Doppelbindung bedeutet, sowie das Gemisch der drei isomeren Acetylcycloundecene der allgemeinen Formel II.

2. Verwendung des Acetylcycloundecans und der Acetylcycloundecene nach Anspruch 1 als Riechstoffe.

3. Riechstoffkompositionen, dadurch gekennzeichnet, daß sie das Acetylcycloundecan oder die Acetylcycloundecene nach Anspruch 1 in einer Menge von 1—50 Gewichtsprozent, bezogen auf die gesamte Komposition, enthalten.

**Claims**

1. Acetyl cycloundecane corresponding to formula I and acetyl cycloundecenes corresponding to general formula II

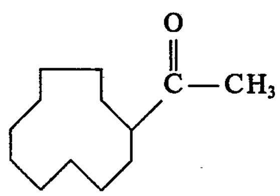

(I)

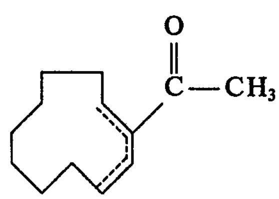

(II)

in which one of the bonds drawn in chain lines is a double bond, and also the mixture of the three isomeric acetyl cycloundecenes corresponding to general formula II.

2. The use of the acetyl cycloundecane and of the acetyl cycloundecenes claimed claim 1 as perfumes.

3. Perfume compositions, characterized in that they contain the acetyl cycloundecane or the acetyl cycloundecenes claimed in claim 1 in a quantity of from 1 to 50% by weight, based on the composition as a whole.

**Revendications**

1. Acétylcycloundécane de formule I et acétylcycloundécènes de formule générale II

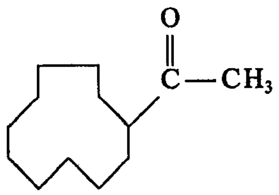

(I)

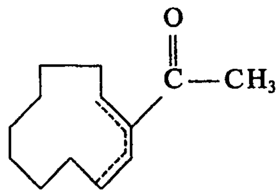

(II)

dans laquelle chaque fois une des liaisons dessinées en tirets signifie une double liaison, ainsi que le mélange des trois acétylcycloundécènes isomères de formule générale II.

2. Utilisation de l'acétylcycloundécane et des acétylcycloundécènes selon la revendication 1 en tant que substances odorantes.

3. Compositions de substances odorantes, caractérisées en ce qu'elles contiennent l'acétylcycloundécane ou les acétylcycloundécènes selon la revendication 1 en une quantité de 1 à 50% en poids par rapport à la composition totale.